# EUROPEAN PATENT APPLICATION

(11) **EP 4 293 101 A1**
(43) Date of publication of application: **20.12.2023**
(21) Application number: 22191879.0
(22) Date of filing: 24.08.2022
(51) Int. Cl.: C12M 1/00, C12M 3/06, H05B 3/26, C12M 1/02

(54) **REACTOR WITH TEMPERATURE CONTROL AND METHOD OF USING THE SAME**

(30) Priority: 14.06.2022 EP 22382567
(71) Applicant: Asklepios Biopharmaceutical, Inc., Research Triangle Park, NC 27709 (US)
(72) Inventor: Martínez Mogarra, Alfredo, 20010 Paseo de Errondo 10 1 A. San Sebastian. Gipuzkoa (ES); Martín Parrón, Sergio, 20009 Camino de San Juan de Dios 7. San Sebastián. Guipuzkoa (ES); Bastida-Corcuera, Felix D., 20006 Autonomia 11-1Esk. San Sebastian,Guipuzkoa (ES)
(74) Representative: Herrero & Asociados, S.L.

(57) **Abstract**

A reactor includes a support base, at least one wall, a top cover and a moveable plate assembly. The top cover, the support base and the at least one wall form an internal chamber therein. The moveable plate assembly includes a moveable plate located within the internal chamber. The moveable plate being configured to move in the x-direction, y-direction, and the z-direction. The temperature of the moveable plate is controllable to a desired temperature. The internal chamber is accessible via at least one of the top cover, the support base, and the at least one wall.

## Description

### FIELD OF THE INVENTION

This application is directed to reactors or bioreactors and, more specifically, is directed to reactors to be used in reactions that require temperature control and/or controlled homogenous mixing.

### BACKGROUND OF THE INVENTION

Reactors or bioreactors have been used in the past in enzymatic reactions. These reactors are used in cell culture (eukaryotic or prokaryotic cells). These reactors are not user friendly for larger volumes, and often also involve steps such as manually shaking the bag containing the material from time to time. Manual steps such as shaking are often disadvantageous in reactions, especially those reactions involving larger strands that may be prone to potentially nicks that will break the longer chains. If the material is viscous and shook with improper mechanical forces, the chains can break down, resulting in reduced production yields. This can happen frequently with, for example, DNA. It would be desirable to have a reactor that addresses these disadvantages of existing reactors, while still being of a desired industrial scale and flexibility in the manufacturing methods.

### SUMMARY

According to one aspect of the present disclosure, a reactor comprises a support base, at least one wall, and a top cover. The top cover, the support base and the at least one wall form an internal chamber therein. The reactor further comprises a moveable plate assembly including a moveable plate located within the internal chamber. The moveable plate is configured to move in the x-direction, y-direction, and the z-direction. The temperature of the moveable plate is controllable to a desired temperature. The internal chamber is accessible via at least one of the top cover, the support base, and the at least one wall.

According to a configuration of the above implementation, the top cover, the support base, and the at least one wall comprise stainless steel.

According to a configuration of the above implementation, the at least one wall forms a porthole for viewing into the internal chamber. In one implementation, the at least one wall is a plurality of walls and the plurality of walls forms a respective porthole for viewing into the internal chamber.

According to another configuration of the above implementation, the internal chamber is accessible by moving a portion of the at least one wall and a portion of the top cover via a hinge.

According to a further configuration of the above implementation, the moveable plate comprises aluminum.

In a further aspect of the above implementation, the moveable plate comprises a plurality of layers. In one implementation, the moveable plate comprises a resistive heating element layer.

In a further aspect of the above implementation, the maximum inclination of the moveable plate is about 20 degrees or about 30 degrees in any direction.

In yet a further aspect of the above implementation, the movement of the moveable plate uses linear movement, rotary movement and orbital movement.

According to another aspect of the present disclosure, the reactor has a width of from about 500 mm to about 1,500 mm, a height of from about 500 mm to about 2,000 mm, and a depth of from about 500 mm to about 1,500 mm.

According to a configuration of the above implementation, further including filters to assist in preventing or inhibiting cross-contamination.

According to another configuration of the above implementation, the at least one wall is a plurality of walls.

According to a further configuration of the above implementation, wherein the internal chamber is hermetically closed.

According to another aspect of the present disclosure, a reactor is provided that comprises a support base, at least one wall, and a top cover. The top cover, the support base and the at least one wall form an internal chamber therein. The reactor further comprises a moveable plate assembly including a moveable plate located within the internal chamber. The moveable plate is configured to move in the x-direction, y-direction, and the z-direction. The temperature of the moveable plate is controllable to a desired temperature. The internal chamber is accessible via at least one of the top cover, the support base, and the at least one wall. A bag or package is placed on the moveable plate in a secured position. The bag includes at least one reaction component and an enzyme. The moveable plate is heated to a desired temperature. The temperature is controlled within the internal chamber. The moveable plate is moveable in the x-direction, y-direction and the z-direction so as to mix the at least one reaction component and the enzyme.

According to a configuration of the above implementation, the bag is from about 1 to about 20 liters.

According to a configuration of the above implementation, the at least one reaction component is a nucleic acid. The nucleic acid may be DNA and/or RNA polymerization.

According to another configuration of the above implementation, the at least one reaction component least one reaction component and an enzyme involves restriction digestion with restriction endonucleases; nucleic acid ligation; reactions with telomerase or protelomerase; reactions with endonuclease and/or exonuclease; reactions with DNA polymerase or any variant thereof; reactions with reverse transcriptase; reactions with recombinase, reaction with protease; reaction with nuclease; polymerase chain reaction, nucleic acid amplification; phosphorylation reaction; dephosphorylation reaction; nucleic acid synthesis reaction; reaction with RNA polymerase; nick or damage repair reaction of nucleic acid; nucleic acid denaturation reaction; nucleic acid annealing reaction; nucleic acid extension reaction; or any combination thereof.

According to a further configuration of the implementation, the at least one reaction component and an enzyme involves restriction endonucleases; nucleic acid; amino acid; ligase; telomerase or protelomerase; deoxynucleoside triphosphate (dNTPs); endonuclease and/or exonuclease; DNA polymerase or any variant thereof; reverse transcriptase; recombinase, protease; nuclease; Taq DNA polymerase,; phosphorylase; phosphotransferase; dephosphorylase; nucleic acid synthase; RNA polymerase; or any combination thereof.

According to yet another embodiment, the enzymatic reaction is restriction digestion with restriction endonucleases; nucleic acid ligation; reaction with telomerase or protelomerase; reaction with endonuclease and/or exonuclease; reaction with DNA polymerase or any variant thereof; reaction with reverse transcriptase; reaction with recombinase, reaction with protease; reaction with nuclease; polymerase chain reaction, nucleic acid amplification reaction; phosphorylation reaction; dephosphorylation reaction; nucleic acid synthesis reaction; reaction with RNA polymerase; reaction of nick or damage repair of nucleic acid; nucleic acid denaturation reaction; nucleic acid annealing reaction; nucleic acid extension reaction; or any combination thereof.

The above summary is not intended to represent each embodiment or every aspect of the present invention. Additional features and benefits of the present invention are apparent from the detailed description and figures set forth below.

### BRIEF DESCRIPTION OF DRAWINGS

Other advantages of the invention will become apparent upon reading the following detailed description and upon reference to the drawings in which:
FIG. 1 is a generally front and side perspective view of a reactor or a bioreactor according to one embodiment.
FIG. 2A is a top perspective view of a moveable plate according to one embodiment that is used in the reactor of FIG. 1.
FIG. 2B is an enlarged view of the moveable plate used in FIG. 2A.
FIG. 2C is a moveable plate according to another embodiment.
FIG. 3 is a top perspective view of a resistive heating element layer used in the moveable plate of FIGS. 2A, 2B according to one embodiment.
FIG. 4A is a bottom perspective view of a moveable plate assembly, including the moveable plate of FIG. 2A, in one position.
FIG. 4B is a side perspective view of the moveable plate assembly of FIG. 4A in another position.
FIG. 5A is a top perspective view of a moveable plate assembly including an adjustable locking system according to one embodiment.
FIG. 5B is a top perspective view of a moveable plate assembly including an adjustable locking system according to another embodiment.
FIG. 6 is a back perspective view of a mechanical movement system to move the moveable plate assembly of FIGS. 4A and 4B according to one embodiment.
FIG. 7A is a side view of a reactor according to another embodiment.
FIG. 7B is a front view of the reactor of FIG. 7A.
FIG. 7C is a partial side perspective view of the reactor of FIG. 7A.
FIG. 8 is an exploded view of a flow sensor to measure viscosity according to one embodiment.
FIG. 9A is a bag or package that may be used with a reactor according to one embodiment.
FIG. 9B is a bag or package secured to the moveable plate assembly of FIG. 5B according to another embodiment.

While the invention is susceptible to various modifications and alternative forms, specific embodiments thereof have been shown by way of example in the drawings and will herein be described in detail. It should be understood, however, that it is not intended to limit the invention to the particular forms disclosed, but on the contrary, the intention is to cover all modifications, equivalents, and alternatives falling within the spirit and scope of the invention as defined by the appended claims.

### DETAILED DESCRIPTION

This application in one embodiment is directed to a reactor. The reactor includes a support base, at least one wall, a top cover, and a moveable plate assembly. The top cover, the support base, and the at least one wall form an internal chamber therein that is hermetically closed. The moveable plate assembly includes a moveable plate located within the internal chamber. The moveable plate is configured to move in the x-direction, y-direction, and the z-direction. The temperature of the moveable plate is controllable to a desired temperature. The internal chamber of the reactor is accessible via at least one of the top cover, the support base, and the at least one wall. In one embodiment, the reactor is an incubation system capable of mixing materials (e.g., genetic material) and maintaining it at a desired or required temperature.

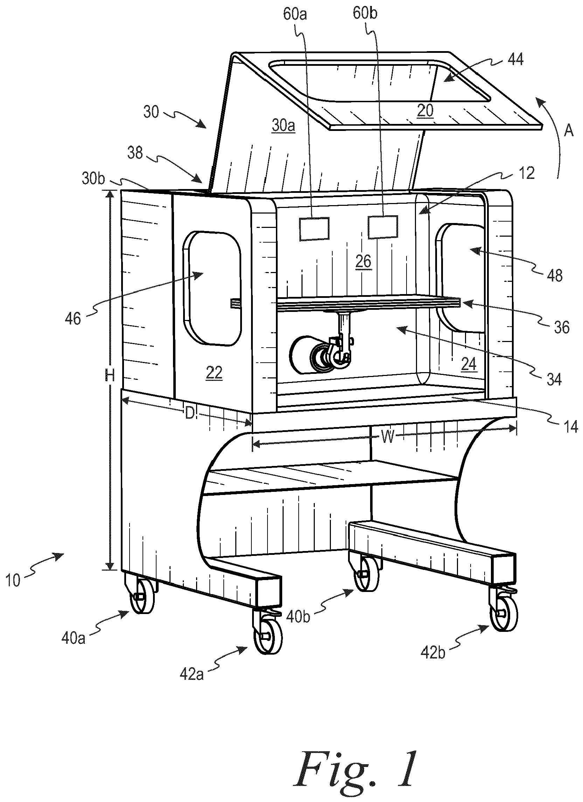

Referring to FIG. 1, a reactor or bioreactor 10 is shown according to one embodiment. The reactor 10 is in an open position such that a user can access an internal chamber 12 of the reactor 10. The reactor 10 includes a support base 14, a plurality of walls 20, 22, 24, 26, a top cover 30, and a moveable plate assembly 34. The combination of the plurality of walls 20, 22, 24, 26, the top cover 30, and the support base 14 form the internal chamber 12 therein, which is hermetically closed and sealed in this embodiment. Thus, the reactor is a fully isolated apparatus from the surrounding environment in one embodiment. The reactor 10 has a depth D, a width W and a height H.

The internal chamber 12 of FIG. 1 is accessible by moving the top cover 30 and the front wall 20. In this embodiment, the top cover 20 and the front wall 30 are integrally formed. More specifically, the internal chamber 12 is accessible by moving or pivoting the front wall 20 and a first portion 30a of the top cover 30 via a hinge 38 along the general path of arrow A to an open position. This type of opening of the reactor to access the internal chamber 12 may be referred to as an ascending opening. A second portion 30b of the top cover 30 remains stationary when the internal chamber 12 is being accessed. It is contemplated that an internal chamber of a reactor may be accessed by other methods. For example, accessing of the internal chamber may involve movement of one or more of the front wall, sidewalls, back wall and/or top cover.

The shape of the internal chamber 12 of the reactor 10 in FIG. 1 is generally rectangular. It is contemplated that an internal chamber of a reactor may be other polygonal shapes such as square, a pentagon, a hexagon or octagon. It is also contemplated that the shape of an internal chamber of a reactor may be non-polygonal such as generally oval or circular. It is contemplated that the shape of the internal chamber may be of other polygonal and non-polygonal shapes than specifically mentioned above.

The reactor 10 as shown in FIG. 1 includes a plurality of wheels 40a, 40b, 42a, 42b at a bottom thereof. The wheels 40a, 40b are in a fixed position, while the wheels 42a, 42b are mobile wheels. The plurality of wheels 40a, 40b, 42a, 42b assists in easily moving the reactor 10. It is contemplated that a reactor may be designed with different wheels or no wheels at all.

At least one of the plurality of walls may form a porthole for viewing into the internal chamber. Referring still to FIG. 1, the front wall 20 forms a porthole 44, the side wall 22 forms a porthole 46, and the side wall 24 forms a porthole 48. The portholes 44, 46, 48 may be double-glazed portholes with ultraviolet (UV) protection. In such an embodiment, the portholes 44, 46, 48 allow desirable visibility of the process occurring in the internal chamber 12 of the reactor 10. It is contemplated that the walls of a reactor may include more or less portholes than shown in FIG. 1, including no portholes.

The moveable plate assembly 34 includes a moveable plate 36. The moveable plate 36 is located within the internal chamber 12 of the reactor 10 of FIG. 1. The moveable plate 36 includes a plurality of layers in this embodiment. It is contemplated that a moveable plate may be made of a single layer in another embodiment.

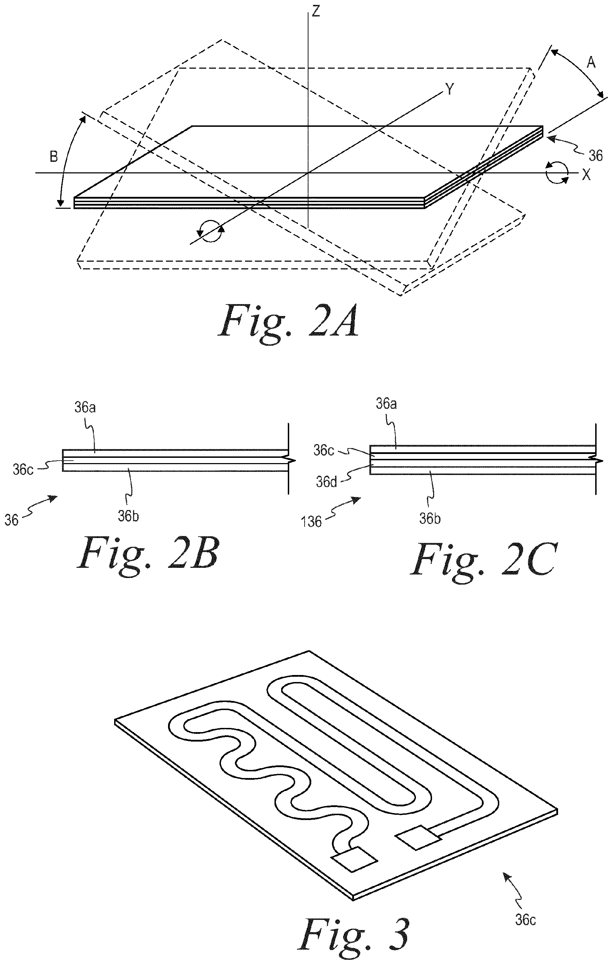

FIG. 2A shows a top perspective view of a moveable plate 36 with potential locations, while FIG. 2B is an enlarged view of a portion of the moveable plate 36 of FIG. 2A showing its individual layers. As shown best in FIG. 2A, the moveable plate 36 is configured to move in the x-direction, y-direction, and the z-direction. The maximum inclination of the moveable plate 36 is shown as angles A, B in FIG. 2A. In one embodiment, the maximum inclination of the moveable plate 36 is about 20 degrees in any direction. In another embodiment, the maximum inclination of the moveable plate 36 is about 25 degrees in any direction. In a further embodiment, the maximum inclination of the moveable plate 36 is about 30 degrees in any direction. The inclinations of the moveable plate 36 is generally from about 5 to about 25 degrees in any direction. In other embodiments, the inclinations of the moveable plate are from about 10 to about 25, or from about 10 to 20 degrees in any direction.

It is contemplated that the maximum inclinations can vary between the x-direction, y-direction, and the z-direction. It is also contemplated that the maximum inclinations can be the same in the x-direction, y-direction, and the z-direction.

FIG. 2B shows the moveable plate 36 including a first moveable plate layer 36a, a second moveable plate layer 36b, and a resistive heating element layer 36c. The resistive heating element layer 36c is located between the first and second moveable plate layers 36a, 36b.

It is also contemplated that additional layers may be included in the moveable plate such as a silicon layer. If a silicone layer is used, it is typically directly adjacent to the resistive heating element layer 36c. One non-limiting example is shown in FIG. 2C with a moveable plate 136. The moveable plate 136 includes the first moveable plate layer 36a, the second moveable plate layer 36b, the resistive heating element layer 36c and a silicone layer 36d. The resistive heating element layer 36c is located between the first moveable plate layer 36a and the silicone layer 36d. The silicone layer 36d is located between the resistive heating element layer 36c and the second moveable plate layer 36b. It is also contemplated that additional layers may be included in the moveable plate.

The first and second moveable plate layers 36a, 36b in one embodiment comprise aluminum. The aluminum may be treated such as with an anodic oxidation treatment. Aluminum is especially desirable because of its properties includes heat transfer. It is contemplated that the first and second moveable plate layers may comprise other materials such as steel.

Referring to FIGS. 2B, 2C and 3, the resistive heating element layer 36c is configured to heat the moveable plate 36. The resistive heating element layer 36c has electrical current that passes therethrough that generates heat due to the resistive nature of the element's design. Resistive heating element layers may comprise metallic alloys, ceramic materials, or ceramic metals.

The temperature of the moveable plate 36 is controllable to a desired temperature in this embodiment. In one embodiment, the temperature of the moveable plate 36 heated by the resistive heating element layer 36c is controlled by a plurality of calibrated probes (not shown). One non-limiting example of a calibrated probe that may be used to control the temperature of the moveable plate is a PT-100 probe. One probe is typically used for a smaller bag or package, while two probes are typically used for larger bags or packages. It is contemplated that other probes and methods of controlling the temperature of the moveable plate may be used. It is also contemplated that a further probe can be used with an upper temperature limit that can be configured to send an alarm and stop the reactor when the temperature has exceeded this upper temperature limit.

The moveable plate 36 in one embodiment is adapted to be heated up to about 100°C. The moveable plate 36 in another embodiment is adapted to be heated up to about 120°C. The moveable plate 36 in a further embodiment is adapted to be heated up to about 135°C.

In another embodiment, the moveable plate may have a cooling plate layer instead of or in addition to a resistive heating element layer. In such an embodiment, the moveable plate would be cooled instead of heated. If both cooling and heating is desired, water heat exchangers may be used in one embodiment. Hot water can be produced with either a heat pump or resistive heating element (for hot water), which cold water can be produced by Peltier cooler. In this embodiment, the temperature of the moveable plate may be controlled by similar techniques as described above.

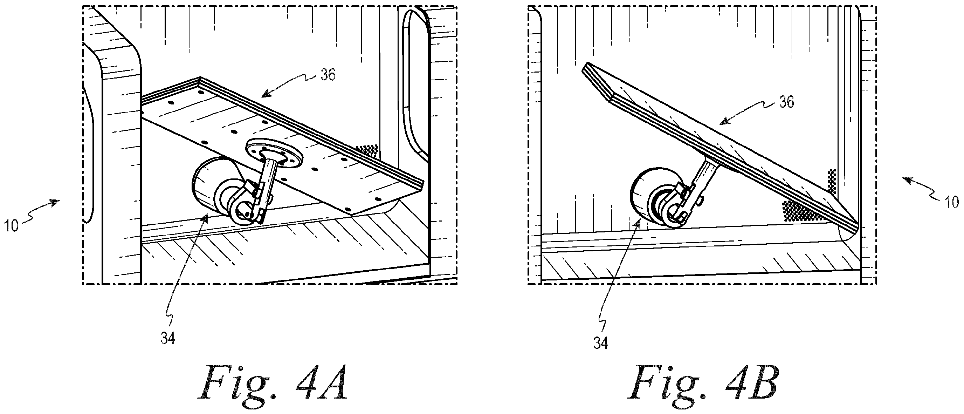

FIGS. 4A, 4B show the moveable plate assembly 34 including the moveable plate 36. FIG. 4A shows the moveable plate 36 in one position within the reactor 10, while FIG. 4B shows the moveable plate 36 in another position within the reactor.

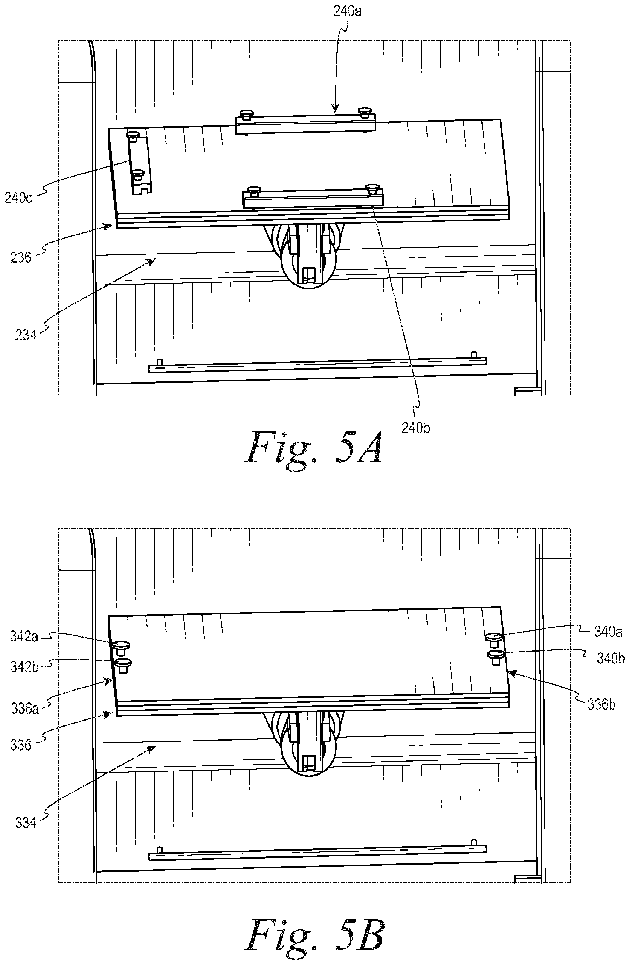

FIG. 5A is a top perspective view of a moveable plate assembly 234 that includes a moveable plate 236 including an adjustable locking system according to one embodiment. The moveable plate 236 is the same as the moveable plate 36 described in detail above, except for the added adjustable clamps 240a, 240b, and 240c. It is contemplated that the adjustable locking system may also be an adjustable locking and sealing system.

The adjustable clamps 240a, 240b, and 240c secure or hold a bag or package on the moveable plate thereon in one embodiment. The adjustable clamps 240a, 240b, and 240c are designed and positioned to work with bag or packages of different sizes. The adjustable clamps 240a, 240b, and 240c are located generally on three sides of the moveable plate 236. More specifically, the adjustable clamps 240a, 240b, and 240c are located near or at the periphery of the moveable plate 236. As shown in FIG. 5A, an adjustable clamp is not typically located on a side of a bag of package where components are added during the reaction. For example, the bag or package may have a liquid component(s) entering the bag or package via tubes.

It is contemplated that fewer or more adjustable clamps may be used than depicted in FIG. 5A. It is also contemplated that a locking or securing system may be implemented that does not involve adjustable clamps.

For example, FIG. 5B shows a top perspective view of a moveable plate assembly 334 that includes a moveable plate 336 according to one embodiment. The moveable plate 336 is the same as the moveable plate 36 described in detail above, except for the added pairs of knobs 340a, 340b and 342a, 342b. The knobs may be designed to be fixed in one embodiment or may be adjustable in another embodiment. The knobs may also be used in combination with one or more brackets that may be adjustable to secure the bag or package.

The pairs of knobs 340a, 340b and 342a, 342b assists in securing or holding a bag or package on the moveable plate thereon in one embodiment. The pairs of knobs 340a, 340b and 342a, 342b are designed and positioned to work with bag or packages of different sizes, especially if they are adjustable and/or work in combination with one or more brackets. The respective pairs of knobs 340a, 340b and 342a, 342b are located next to each other at opposing ends 336a, 336b of the moveable plate 336. More specifically, the pairs of adjustable knobs 340a, 340b and 342a, 342b are located near or at the periphery of the moveable plate 336.

The moveable plates 36, 136, 236, 336 may be moved by different mechanical components and techniques. The movement of the moveable plate uses both linear movement and rotary movement in one embodiment. The movement of the moveable plate is produced through the interpolarization of linear and rotary movements. In another embodiment, the movement of the moveable plate is produced through the interpolarization of linear, rotary and orbital movements. The oscillations of the moveable plate assembly can vary and are set by a user. The oscillations are generally under about 60 oscillations per minute and, typically, under about 45 oscillations per minute and, more specifically, under 30 oscillations per minute. The oscillations typically range from about 5 to about 50 oscillations per minute, and in more typically are from about 10 to about 40 oscillations per minute.

The phase delay degrees of the moveable plate may also vary. The phase delay degrees may be 0 degrees, 90 degrees or 180 degrees.

The movement of the moveable plate can be setup as a mix of different angles, speeds and phase delays to obtain multiple different shaking modes. For example, the phase delay between both axis x and y allows different shakings as orbital, diagonal and mix shakings.

In this embodiment, the movement of the moveable plate is controlled by software including absolute encoders that provide a unique position value at every point of rotation representing the "absolute" position of the encoder. This allows control of the exact position of the rotating shaft in each moment to adjust the inclination and speed selected in moving the moveable plate.

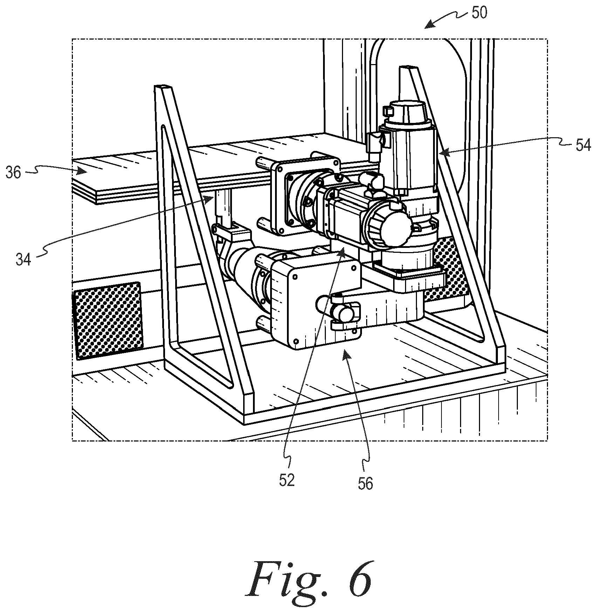

One non-limiting example of a mechanism that can move the moveable plate 36 of the moveable plate assembly 34 is shown in FIG. 6. This mechanism can also move the moveable plates 136, 236, 336 described above.

FIG. 6 shows a mechanical movement system 50 that includes a first brushless motor 52 and a second brushless motor 54. The first and second brushless motors 52, 54 assist in continuously controlling the positions and speed of the moveable plate 36. The first brushless motor 52 is coupled to a pulley and belt system 56 that allows rotary movement. The second brushless motor 54 is coupled to a lever movement that allows linear movement (Y axe inclination). Brushless motors are desirable because of their high power density, good speed-torque characteristics, high efficiency, wide speed ranges, and low maintenance. It is contemplated that other motors may be used to move the moveable plate.

The reactor or bioreactor 10 in one embodiment may further include at least one filter to assist in preventing or inhibiting cross-contamination. A plurality of filters 60a, 60b is shown in FIG. 1 in which the plurality of filters 60a, 60b is mounted on the back wall 26 in the internal chamber 12. A non-limiting example of an air filter that may be used is a HEPA H14 filter. It is contemplated that other filters may be used in the reactor for air filtration.

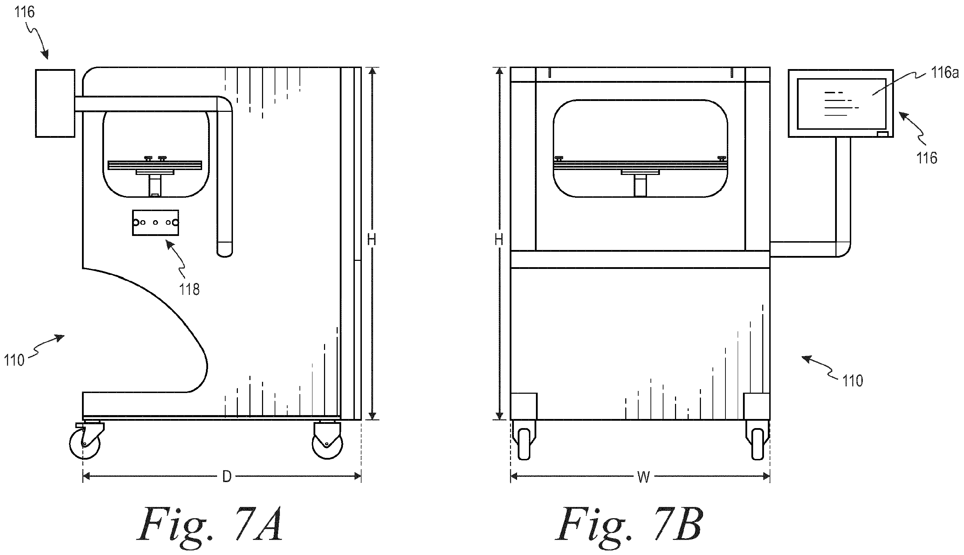

It is contemplated that additional components may be added to the reactor 10. For example, referring to FIGS. 7A-7C, a reactor 110 is shown according to another embodiment. The reactor 110 is substantially the same as the reactor 10 described above, but further includes a monitor 116 with a control panel or screen 116a. The control panel 116a controls the conditions within the reactor 110 including: (1) the temperature conditions of the moveable plate; (2) the temperature of the reactor; and (3) the movement of the moveable plate including the angular movement and the speed of the same. The control panel 116a also controls: (1) the timing of any temperature modifications; and (2) how long and when the movement of the moveable plate assembly occurs. The control panel 116a may control the reactor for a few hours or for several days, if not longer. The control panel 116a may be controlled by touchscreen icons on the screen itself in one embodiment. It another embodiment, the control panel 116a may be controlled by physical buttons or other control mechanisms. It is also contemplated that the control panel in another embodiment may be controlled by a separate computer.

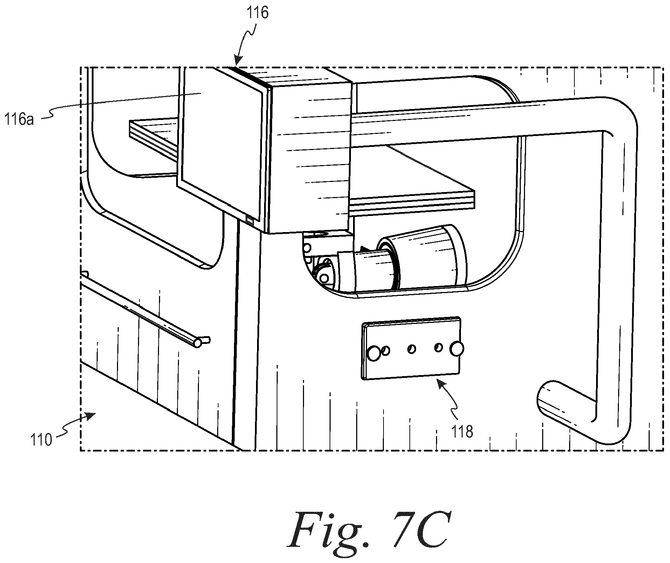

As discussed above, the reactor may be configured to allow liquid to enter the internal chamber during the process. If this is desired, then the reactor may further include a mounted liquid control system with at least one external pump to assist in supplying liquid during the process. The liquid control system allows fluid to exit and re-enter the bag or package located in internal chamber. In one embodiment, the at least one external pump (e.g., a peristaltic pump) transports liquid through flexible tube or hose connections that are connected through a pass-through system (e.g., grommets) in a sidewall. This pass-through system 118 is shown in FIGS. 7A, 7C. One non-limiting example of a peristaltic pump is made by Watson Marlow. This type of pump is desirable because of its ability to be cleaned easily, while still functioning to control the flow of liquid to and from the bag or package. It is contemplated that other pumps may be used.

It is also contemplated that liquid may enter into the bag or package manually during a pause or break in the process steps. In such a process, a user would open the reactor 10 by moving the top cover 30 and the front wall 20 and manually place the liquid into the bag or package.

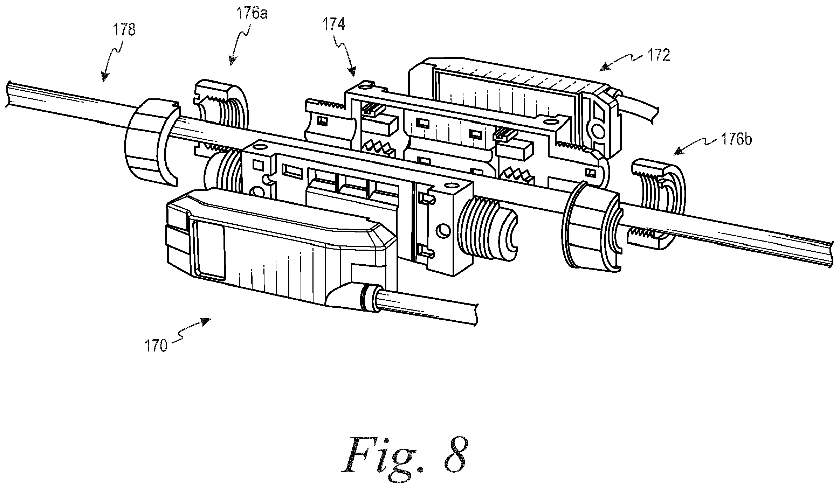

The reactor may further include a viscosity monitoring system to measure and report the viscosity of the components in the bag or package. This may be accomplished by different techniques. For example, a flow sensor may be mounted to a tube where the product itself is made to flow. One non-limiting example of a flow sensor 170 is shown in FIG. 8. The flow sensor 170 includes a sensor head (transceiver) 172, a clamp 174, and a plurality of clamps 176a, 176b. The flow sensor 170 is adapted to be mounted onto a flexible tube 178. One non-limiting commercial example of a flow sensor is the Keyence FD-X envelope flow sensor.

The flow sensor detects the flow velocity in the one or more tubes by crossing them with an ultrasonic beam that will assume different crossing speeds depending on the detected speeds. Here, the velocity will remain constant, but as the viscosity changes, the ultrasound will have a progressively different way of passing through the tubes during the reactor process. The flow sensor does not contact the liquid itself.

In another embodiment, the viscosity changes may be measured by applying Poiseuille law. For example, a peristaltic pump recirculates liquid in the bag or package through a hose or pipe of a certain dimension with a laminar flow (i.e., flow having a Reynolds number (RE)<2300). Two pressure transducers measure the head loss in the hose or pipe. The laminar head loss and viscosity have a linear relationship with each other. This method may be initially calibrated with pure water at 20°C to measure the viscosity first and then with different carbopol solutions. This method is an inexpensive and fairly accurate method to measure viscosity changes.

The dimensions of the reactor 110 are the same the dimensions of the reactor 10. Specifically, the reactors 10, 110 include the depth D, the width W and the height H. In one embodiment, the reactors 10, 110 have a width W of from about 500 mm to about 1,500 mm, a height H of from about 500 mm to about 2,000 mm, and a depth D of from about 500 mm to about 1,500 mm. In one illustrated embodiment, the width is about 1,100 mm, the height is about 1,500 mm, and the depth is about 1080 mm.

The reactors 10, 110 may be made of different materials. According to one embodiment, the reactor is made of metallic material such as stainless steel. More specifically, the top cover, the support base, and the at least one wall comprise stainless steel. It is contemplated that other materials may be used in forming the top cover, the support base, and the wall(s).

### Methods

The methods of the present invention are advantageous in reactions that require temperature control and/or controlled homogenous mixing. These methods avoid disadvantages that can occur from manually shaking the bag or package, while still producing desirable product and yield.

A method of using a reactor for an enzymatic reaction includes providing a reactor. The reactor includes a support base, at least one wall, a top cover and a moveable plate assembly. The top cover, the support base, and the at least one wall form an internal chamber therein that is hermetically closed. The moveable plate assembly including a moveable plate is located within the internal chamber. The moveable plate assembly is configured to move in the x-direction, y-direction, and the z-direction. The internal chamber of the reactor is accessible via at least one of the top cover, the support base, and the at least one wall. The bag is placed on the moveable plate assembly in a secured position. The bag includes at least one reaction component and an enzyme. The moveable plate is heated to a desired temperature. The temperature is controlled within the internal chamber. The moveable plate is moved in the x-direction, y-direction and the z-direction so as to mix the at least one reaction component and the enzyme.

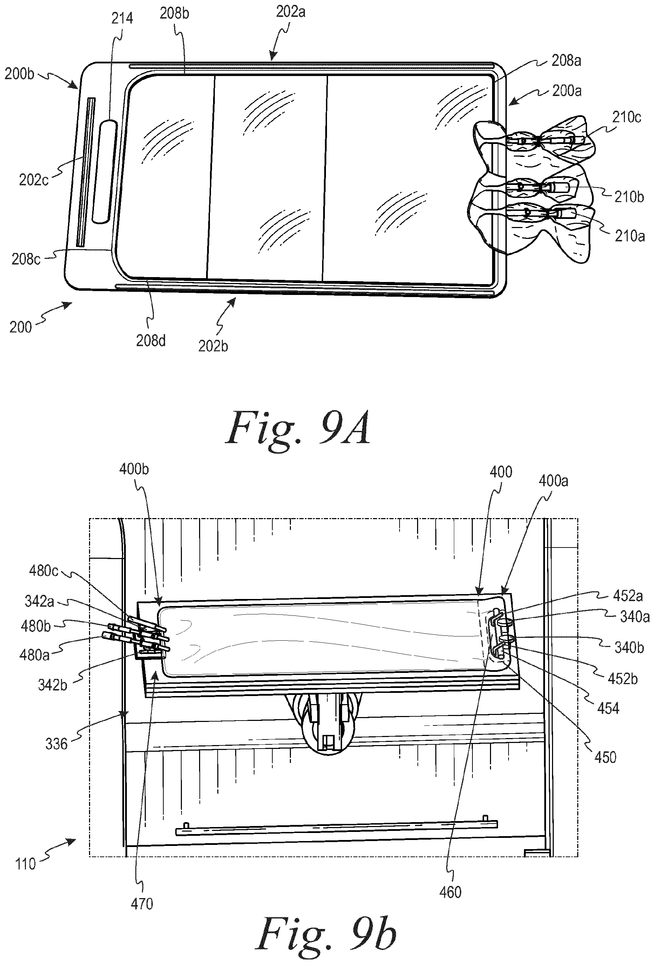

One example of a bag or package 200 that may be used in the reactors 10, 110 is shown in FIG. 9A. This bag or package 200 may be described as a bioprocess container (BPC). The bag or package 200 may vary in size, but typically is from about 1 to about 20 liters. It is contemplated that the bag or package may be smaller than 1 liter or may be larger than 20 liters, including up to 50 liters. The bag 200 is shown as being generally rectangular in size. It is contemplated that the bag or package may be of other shapes and sizes. The bag or package is desirably sized and shaped to remain on the moveable plate during the reaction process, including the mixing.

The bag or package 200 of FIG. 9A includes two elongated fixing sticks 202a, 202b and a shortened stick 202c. These sticks 202a-202c assist in securing the bag 200 to the moveable plate assembly. The bag of package 200 could be used, for example, with the moveable plate assembly 234 of FIG. 5A. It is contemplated that less sticks may be used for securing the bag. It is also contemplated that the length of the sticks may vary.

The bag or package 200 has first end 200a and a second opposing end 202b. At the first end 200a, a plurality of injectors or syringes 210a-210c are shown. The injectors or syringes 210a-210c are luer lock injectors or syringes in one embodiment. It is contemplated that the injectors or syringes may be other types. The injectors or syringes 210a-210c enables additional components to be added to the package 200 during the process. Thus, the injectors or syringes 210a-210c are a conduit for these additional components to be directly entered into the interior of the bag or package 200. The interior of the bag or package 200 does not extend to the sticks 202a-202c, but are sealed along a perimeter identified in FIG. 9A by perimeter sections 208a-208d.

The shortened stick 202c and an aperture 214 are shown at the second end 200b. The combination of the shortened stick 202c and an aperture 214 with a bracket fixture (not shown) assist in securing the bag or package to a moveable plate. The elongated fixing sticks 202a, 202b extend generally between the first end 200a and the second opposing end 202b.

Referring to FIG. 9B, a bag or package 400 is shown in the reactor 110. The bag 400 has a first end 400a and a second end 400b. The bag or package 400 is secured on the moveable plate 336 using the pair of knobs 340a, 340b and 342a, 342b. The pair of knobs 340a, 340b includes an adjustable bracket 450 secured thereto. The adjustable bracket 450 includes a pair of upwardly and outwardly extending hooks 452a, 452b. The pair of upwardly and outwardly extending hooks 452a, 452b extends through an aperture 460 formed at the end 400a of the bag 400. The hooks 452a, 452b in combination with the pair of knobs 340a, 340b secure a fixing stick 454 located inside an opening of the bag 400. The opening is located adjacent to the aperture 460.

The pair of knobs 342a, 342b at the second end 400b includes an adjustable bracket 470 secured attached thereto. The second end 400b of the bag or package 400 is secured underneath of the adjustable bracket 470. At the second end 400b of the bag or package 400, a plurality of injectors 480a-c are shown. These contain components to be added to the bag or package 400.

The reactors of the present invention may carry out biochemical and/or chemical processes that require temperature control and/or controlled homogeneous mixing. It is contemplated that other gases may be used in the reactor to potentially improve enzymatic reactions. One non-limiting example of a gas that may be used is nitrogen.

The reactor or bioreactor may assist in a portion of the biochemical and/or chemical processes such as before the purification steps. Non-limiting examples of biochemical processes include enzymatic process. Some non-limiting enzymatic processes include: restriction digestion with restriction endonucleases; nucleic acid (DNA and/or RNA polymerization); nucleic acid ligation (e.g., DNA ligation); reactions with telomerase or protelomerase; reactions with endonuclease and/or exonuclease; reactions with DNA polymerase or any variant thereof (e.g., with Klenow fragment); reactions with reverse transcriptase; reactions with recombinase, reaction with protease; reaction with nuclease; polymerase chain reaction; nucleic acid (e.g., DNA, complementary DNA, RNA) amplification; phosphorylation reaction; dephosphorylation reaction; nucleic acid synthesis reaction (e.g., oligonucleotide synthesis or primer synthesis); reaction with RNA polymerase; nick or damage repair reaction of nucleic acid; nucleic acid denaturation reaction; nucleic acid annealing reaction; nucleic acid extension reaction; or any combination thereof.

In some embodiments, the enzymatic reaction that are carried out by the bioreactor of the invention uses any nucleic acid known in the art as a substrate of the enzyme. In some embodiments, the enzymatic reaction that is performed in the bioreactor uses any protein or, polypeptide known in the art as a substrate of the enzyme. In one aspect of the embodiment, the protein or polypeptide substrate is an antibody or a fragment thereof. In one embodiment, the nucleic acid modification (e.g., mutation, substitution, insertion, or deletion reaction) is performed using the bioreactor of the invention. In another embodiment, the bioreactor of the invention carries out methylation, demethylation, phosphorylation, dephosphorylation, or glycosylation reaction of nucleic acid and/or protein. In yet another embodiment, glycation of a protein is performed using the bioreactor of the invention. In some embodiments of the invention, the bioreactor is used for messenger RNA (mRNA) production.

In certain embodiments, the bioreactor of the invention is used to perform the modification of antibody. This may include, for example, antibody enzyme labeling, antibody nanoparticle labeling, antibody fragmentation, antibody biotinylation, antibody liposome conjugation, antibody immobilization or antibody oligonucleotide conjugation.

In some embodiments, the bioreactor of the invention is used for one or more steps of vaccine manufacturing process. Non-limiting examples of vaccine are Influenza vaccine, Covid-19 vaccine, Hepatitis A vaccine, Hepatitis B vaccine, HPV vaccine, Polio vaccine, Rabies vaccine, diptheria vaccine, Rotavirus vaccine, Rubella vaccine, shingles vaccine, smallpox vaccine, tetanus vaccine, Meningococcal vaccine, Varicella vaccine, and Pneumococcal vaccine.

In some embodiments, the bioreactor of the invention is used to perform gene editing reaction using any one of meganucleases, zinc finger nucleases (ZFNs), transcription activator-like effector-based nucleases (TALEN), and the clustered regularly interspaced short palindromic repeats (CRISPR/Cas9) system. The gene editing reaction repairs a double strand break in DNA by non-homologous end joining (NHEJ) or by homology directed repair (HDR), and thus either creates gene disruption by insertion or deletion, or create precise DNA editing. In certain embodiments, the bioreactor of the invention is used to perform conditional targeting of genes using site specific recombinases (e.g., Cre-loxP or Flp-FRT).

In some embodiments, the bioreactor of the invention is used for chemical reaction (e.g., in organic chemical synthesis reaction to synthesize small molecules) conjugation of probes using click chemistry, attaching larger macromolecule such as PEGylation, producing azo-compound, and the like. In the aspects of using the bioreactor of the invention for performing chemical reaction that require temperature control and/or homogeneous mixing may or may not need an enzyme as a reaction component.

In some embodiments, the enzymatic reactions that are performed using the bioreactor of the invention is performed at or, above room temperature. For example, the temperature may be at least about 25°C, at least about 30°C, at least about 32°C, at least about 37°C, at least about 40 °C, at least about 50°C, at least about 60°C, at least about 70°C, at least about 90°C, at least about 95°C, at least about 100°C or even higher. In some embodiments, the enzymatic reactions that are performed using the bioreactor of the invention is performed at below room temperature. For example, the temperature may be below about 25°C, below about 20°C, below about 15°C, at below about 10°C, below about 5°C , below about 4°C, or below about 0°C.

Thus, the at least one reaction component in the bag or package is a nucleic acid in some embodiments. Some non-limiting examples of nucleic acids that can be used include DNA, RNA, oligonucleotide, inhibitory nucleic acids (e.g., miRNA, siRNA, RNAi, shRNA, dsRNA or variants thereof). Some non-limiting examples of DNA include cell free DNA, purified or isolated DNA, nicked or damaged DNA, modified DNA (e.g., DNA comprising substitution, insertion, deletion or any other modification such as, for example, methylation, demethylation, phosphorylation, dephosphorylation, addition of sugar moiety, conjugation of larger macromolecule such as polyethylene glycol, comprising chemical modification, or conjugation of labels or dyes). Some non-limiting examples of RNA include messenger RNA (mRNA), total RNA, transfer RNA (tRNA), RNA comprising substitution, insertion, deletion or any other modification (e.g., methylation, demethylation, phosphorylation, dephosphorylation, addition of sugar moiety, conjugation of larger macromolecule such as polyethylene glycol, comprising chemical modification, or conjugation of labels or dyes). Some examples of modified DNA and/or RNA include DNA and/or RNA comprising modified bases (e.g., 2'-O-methoxy-ethyl Bases, 2' O Methyl RNA bases, fluoro bases, 2 Aminopurine, 5 bromo dU, deoxyuridine or the like. In some embodiments, the at least one reaction component in the bag or package is a protein, polypeptide or the like. Some non-limiting examples of polypeptide or protein include isolated or purified protein, semi purified protein, proteins or polypeptides that are phosphorylated, dephosphorylated, methylated, demethylated, glycated or glycosylated.

In the aspects of the embodiments described herein, the bioreactor of the invention produces consistent results with minimal variability. Thus it may be suitably used for various biochemical and/or chemical processes where the comparability protocols may be successfully performed minimizing the variability occurring from the temperature variance and/or inconsistent mixing of reaction components.

In some embodiments, the bioreactor of the invention (also referred to as an inventive reactor) minimizes the variability between different batches of a reaction by at least about 25%, by at least about 20%, by at least about 15%, by at least about 10%, by at least about 5%, by at least about 3 %, by at least about 2.5%, by at least about 2%, or by at least about 1 % than when the different batches of the reaction are performed manually. In some embodiments, the bioreactor of the invention minimizes the variability between different batches of a reaction by about 2% to about 10%, about 5% to about 15%, about 10% to about 20%, about 15% to about 25%, about 20% to about 30%, about 25% to about 40% or even more than when the different batches of the reaction are performed manually.

The less variability between batches of a reaction using the bioreactor of the invention has immense value, in particular, for manufacturing clinical products. The regulatory authorities across the globe appreciate less variability between different production batches and, thus, the bioreactor of the invention can help adhere to regulatory compliance of a product that is produced in different batches using the bioreactor of the invention. The non-limiting examples of product may include, for example, close ended linear duplex DNA (clDNA or celDNA) or viral vectors such as AAV vectors manufactured using clDNA as a template. The less variability between batches is also appreciated in manufacturing products on an industrial scale.

In some embodiments, the at least one reaction component in the bag or package is a double-stranded (ds) or a single-stranded (ss) DNA. A double-stranded DNA may be an open circular double-stranded DNA, a closed circular double-stranded DNA, an open linear double-stranded DNA, or a closed linear double-stranded DNA. Preferably, the double-stranded DNA is a closed circular double-stranded DNA.

In some embodiments, the bioreactor is used to synthesize close ended linear duplex nucleic acids. Non-limiting examples of close ended linear duplex nucleic acids that may be produced using the bioreactor of the present invention include doggy bone DNA (dbDNA) or dumbbell-shaped DNA. The close ended linear duplex nucleic acids may be generated within cells or, using *in vitro* cell free system.

Non-limiting examples of methods that may be performed in one or more steps using the bioreactor of the invention include cell free *in vitro* synthesis of dumbbell-shaped DNA and doggy bone DNA as described in U.S. Patent No. 6,451,563; Efficient production of superior dumbbell-shaped DNA minimal vectors for small hairpin RNA expression-Nucleic Acids Res. 2015 Oct 15; 43(18): e120; High-Purity Preparation of a Large DNA Dumbbell-Antisense & nucleic acid drug development 11:149-153 (2001);US 9,109,250; U.S. Patent No. 9,499,847; U.S. Patent No. 10,501,782; and WO 2018033730 A1; all of which are herein incorporated by reference in their entireties. The DNA from cell free *in vitro* synthesis is devoid of any prokaryotic DNA modifications (e.g., is substantially free of bacterial DNA). In some embodiments, the close ended linear duplex nucleic acid synthesized using the bioreactor of the invention has less than 1% bacterial DNA.

One example of an *in vitro* process for producing a closed linear DNA (e.g., containing the ITRs described herein) comprises (a) contacting a DNA template flanked on either side by a protelomerase target sequence with at least one DNA polymerase in the presence of one or more primers under conditions promoting amplification of said template; and (b) contacting amplified DNA produced in (a) with at least one protelomerase under conditions promoting formation of a closed linear expression cassette DNA. The closed linear DNA may be a closed DNA expression cassette DNA product that may comprise, consist or consist essentially of a eukaryotic promoter operably linked to a coding sequence of interest and, optionally, a eukaryotic transcription termination sequence. The closed linear expression cassette DNA product may additionally lack one or more bacterial or vector sequences, typically selected from the group consisting of: (i) bacterial origins of replication; (ii) bacterial selection markers (typically antibiotic resistance genes) and (iii) unmethylated CpG motifs.

As outlined above, any DNA template comprising at least one protelomerase target sequence may be amplified according to the bioreactor of the invention. Thus, although production of therapeutic DNA molecules (e.g., for DNA vaccines or other therapeutic proteins and nucleic acid) is preferred, the bioreactor of the invention may be used to produce any type of closed linear DNA. The DNA template may be a double stranded (ds) or a single stranded (ss) DNA. A double stranded DNA template may be an open circular double stranded DNA, a closed circular double stranded DNA, an open linear double stranded DNA or a closed linear double stranded DNA. The template is desirably a closed circular double stranded DNA. Closed circular dsDNA templates are particularly preferred for use with RCA (rolling circle amplification) DNA polymerases. A circular dsDNA template may be in the form of a plasmid or other vector typically used to house a gene for bacterial propagation. Thus, using the reactor can amplify any commercially available plasmid or other vector, such as a commercially available DNA medicine, and then convert the amplified vector DNA into closed linear DNA.

Phi29 DNA polymerase is used to amplify double-stranded DNA by rolling circle amplification, and a protelomerase to generate close ended linear duplex DNA (clDNA or celDNA), which coupled with a streamlined purification process, results in a pure DNA product containing just the sequence of interest. Phi29 DNA polymerase has high fidelity (e.g., 1×10⁶-1×10⁷) and high processivity (e.g., approximately 70 kbp). These features make this polymerase particularly suitable for the large-scale production of GMP DNA. Protelomerases (also known as telomere resolvases) catalyze the formation of covalently closed hairpin ends on linear DNA and have been identified in some phages, bacterial plasmids and bacterial chromosomes. A pair of protelomerases recognizes inverted palindromic DNA recognition sequences and catalyzes strand breakage, strand exchange and DNA ligation to generate closed linear hairpin ends. The formation of these closed ended structures makes the DNA resistant to exonuclease activity, allowing for simple purification and can improve stability and duration of expression. The bioreactor of the invention is used to perform rolling circle amplification and/or protelomerase reaction, or a combination thereof to generate close ended linear duplex DNA (clDNA or celDNA).

An open circular dsDNA may be used as a template where the DNA polymerase is a strand displacement polymerase that can initiate amplification from at a nicked DNA strand. In this embodiment, the template may be previously incubated with one or more enzymes which nick a DNA strand in the template at one or more sites. A closed linear dsDNA may also be used as a template. The closed linear dsDNA template (starting material) may be identical to the closed linear DNA product. Where a closed linear DNA is used as a template, it may be incubated under denaturing conditions to form a single stranded circular DNA before or during conditions promoting amplification of the template DNA.

In one example, the reactor or bioreactor as described in this invention is used to perform the rolling circle amplification (RCA) process to generate amplified DNA followed by digestion of the amplified DNA with restriction enzymes, and further contacting the digested form of amplified DNA with at least one protelomerase, in an automated way with controlled temperature and controlled homogeneous mixing conditions and, thus, forming doggy bone DNA (dbDNA). In this example, the template used for RCA may be a nucleic acid such as open circular dsDNA or closed linear dsDNA. The RCA process comprises denaturation of the template DNA followed by contacting with oligonucleotides (serving as primers), dNTP (deoxynucleoside triphosphate), and another enzyme (Phi29 polymerase). In this embodiment, a buffer is typically included.

The duration, speed, angles and phase delay of the moveable plate as well as the incubation temperature of the reactor can vary depending on the reaction. In one non-limiting example of performing a reaction for forming clDNA, the temperature varies from about 25 to 40 degrees Celsius. The incubation times can vary from minutes to hours to get the proper steps completed in a desirable fashion. For example, the RCA step may be incubated from about 25 to 35 hours and the RE step may be from about 10 to about 80 hours. The TelN steps may be incubated from about 15 minutes to about 35 minutes, while the exonucleases processing may be incubated from about 15 to about 25 hours.

The moveable plate in the inventive reactor typically is oscillating at a speed of from about 10 to about 50 rpm with angles from about 10 to about 20 degrees with different phases (0, 90 and 180 degrees) in reactions for forming clDNA.

Thus, in one method, the components include a buffer, a plurality of enzymes, nucleic acid template, deoxynucleotide triphosphates (dNTPs) and at least one primer.

The reactants for specific reactions are delivered to the bag used in the reactor either subsequently to one another, or, in different combinations. After each incubation of the reactions at desired temperature, e.g., after certain time intervals, the reactants of the next reactions are added to the bag.

In certain embodiments the bioreactor of the invention is used to perform one or more steps of alternate methods of generating covalently closed end linear DNA that lack bacterial sequences e.g., by formation of mini-circle DNA from plasmids as described in U.S. Patent No. 8,828,726, and U.S. Patent No. 7,897,380, the contents of each of which are incorporated by reference in their entirety. For example, one method of cell-free synthesis combines the use of two enzymes (Phi29 DNA polymerase and a protelomerase) and generates high fidelity, covalently closed, linear DNA constructs. The constructs contain no antibiotic resistance markers and therefore eliminate the packaging of these sequences.

In some embodiments, the bioreactor of the invention is used to perform one or more steps of generating covalently closed DNA as described in published application U.S. Publication No. 2020/0362403, which is incorporated hereby by reference in its entirety. The bioreactor is used for all of the following steps or, any one or any combination of the following steps that comprise pretreating the linear double-stranded DNA template (e.g., removing the protruding ends; restoring 5'Phosphate and 3'OH groups; adding the 3' dA overhangs; ligating the hairpin adapter at 5' and 3' end followed by forming a single-stranded covalently closed DNA that undergoes rolling circle amplification; and multiple strand displacement by a combination of enzymes Primase-Polymerase and Phi29 Polymerase).

Phi29 DNA polymerase is used to amplify double-stranded DNA by rolling circle amplification, and a protelomerase to generate covalently closed linear DNA, which coupled with a streamlined purification process, results in a pure DNA product containing just the sequence of interest. Phi29 DNA polymerase has high fidelity Phi29 DNA polymerase has high fidelity (e.g., 1×10⁶-1×10⁷) and high processivity (e.g., approximately 70 kbp). These features make this polymerase particularly suitable for the large-scale production of GMP DNA. Protelomerases (also known as telomere resolvases) catalyze the formation of covalently closed hairpin ends on linear DNA and have been identified in some phages, bacterial plasmids and bacterial chromosomes. A pair of protelomerases recognizes inverted palindromic DNA recognition sequences and catalyzes strand breakage, strand exchange and DNA ligation to generate closed linear hairpin ends. The formation of these closed ended structures makes the DNA resistant to exonuclease activity, allowing for simple purification and can improve stability and duration of expression.

In one embodiment, the close ended linear duplex DNA is produced in eukaryotic cells for example insect cells as described in PCT publications WO 2019032102 and WO 2019169233. In one embodiment, the DNA is not produced in eukaryotic cells and DNA lacks eukaryotic sequences. In one embodiment, the close ended liner duplex DNA vectors are produced as described in PCT publication WO 2019143885.

In certain embodiments, an *in vivo* cell system is used to produce close ended linear duplex nucleic acids, where one or more steps of the production method is carried out using the bioreactor. The method comprises using a cell that expresses a protelomerase, such as TelN, or other protelomerase, in which the protelomerase gene is under the control of a regulatable promoter. For example, an inducible promoter such as a small molecule regulated promoter or a temperature sensitive promoter (e.g., a heat shock promoter). After sufficient production of the nucleic acid of interest, or combination thereof, one can allow the protelomerase to be expressed, which will excise the nucleic acid of interest from the template.

In certain embodiments, the *in vivo* cell system is used to produce a non-viral DNA vector construct for delivery of a predetermined nucleic acid sequence into a target cell for sustained expression. The non-viral DNA vector comprises, two DD-ITRs each comprising: an inverted terminal repeat having an A, A', B, B', C, C' and D region; a D' region; and wherein the D and D' region are complementary palindromic sequences of about 5-20 nt in length, are positioned adjacent the A and A' region; the predetermined nucleic acid sequence (e.g. a heterologous gene for expression); wherein the two DD-ITRs flank the nucleic acid in the context of covalently closed non-viral DNA and wherein the closed linear vector comprises a ½ protelomerase binding site on each end.

The system comprises recombinant host cells. Suitable host cells for use in the production system include microbial cells, for example, bacterial cells such as E. coli cells, and yeast cells such as S. cerevisiae. Mammalian host cells may also be used including Chinese hamster ovary (CHO) cell for example of K1 lineage (ATCC CCL 61) including the Pro5 variant (ATCC CRL 1281); the fibroblast-like cells derived from SV40-transformed African Green monkey kidney of the CV-1 lineage (ATCC CCL 70), of the COS-1 lineage (ATCC CRL 1650) and of the COS-7 lineage (ATCC CRL 1651; murine L-cells, murine 3T3 cells (ATCC CRL 1658), murine C127 cells, human embryonic kidney cells of the 293 lineage (ATCC CRL 1573), human carcinoma cells including those of the HeLa lineage (ATCC CCL 2), and neuroblastoma cells of the lines IMR-32 (ATCC CCL 127), SK-N-MC (ATCC HTB 10) and SK-N-SH (ATCC HTB 11). The host cell is designed to encode at least one recombinase. The host cell may also be designed to encode two or multiple recombinases. The term "recombinase" refers to an enzyme that catalyzes DNA exchange at a specific target site, for example, a palindromic sequence, by excision/insertion, inversion, translocation and exchange. Examples of suitable recombinases for use in the present system include, but are not limited to, TelN, Tel, Tel (gp26 K02 phage) Cre, Flp, phiC31, Int and other lambdoid phage integrases, e.g. phi 80, HK022 and HP1 recombinases.

The volume of the bag or package can vary, but are generally from about 50 ml to about 10L or about 20L. It is contemplated that the volume may be smaller or larger. The volume of the bag or package is typically from about 500 ml to about 10L.

The inventive reactor used for incubation and automated agitation during different enzymatic reaction steps may use different scaling. Thus, the volume of the reactions may vary that are performed in the inventive reactor. Some typically volume sizes include 20 ml and 500 ml. It is contemplated that larger volume sizes, including 1500 ml and 5000 ml, may be performed in the inventive reactor. The volume of the reactions performed in the inventive reactor is generally from about 10 ml to about 10L in one embodiment. The volume of the reactions performed in the inventive reactor is from about 10 ml to about 5000 ml in another embodiment. The volume of the reactions performed in the inventive reactor is from about 10 ml to about 2000 ml in a further embodiment. The volume of the reactions performed in the inventive reactor is from about 10 ml to about 1000 ml in a further embodiment. The volume of the reactions performed in the inventive reactor is from about 20 ml to about 500 ml in yet another embodiment.

The temperature of the internal chamber may be heated and controlled by different methods. In one embodiment, the temperature of the internal chamber is heated by a resistive heating element layer mounted on a bottom of the support base. The temperature of the internal chamber is controlled in one embodiment by a plurality of probes located thereon such as, for example, on one of the sidewalls. These plurality of probes may be on opposing sidewalls. The probes used may be the same as described above with respect to the moveable plate. It is contemplated that the temperature of the internal chamber may be heated and controlled by other methods.

If ambient temperature is desired within the internal chamber in another embodiment, a plurality of ducts may carry desired air into the reactor. In one embodiment, the plurality of ducts may each be equipped with a fan. In other embodiment, the temperature of the internal chamber of the reactor may be cooled to a desired temperature. In such an embodiment, a cooling system would need to be added and integrated within the reactor. In one embodiment, the cooling system may be a water-based cooling system. In another embodiment, the cooling system may be an air-based cooling system.

### Examples

Several examples were performed implementing and using the inventive reactor described above with respect to FIGS. 1-4 and 6 that used a locking feature and HEPA H14 filters. The inventive reactor was used for incubation and automated agitation during different enzymatic reaction steps using different scaling (20 ml and 500 ml). These inventive reactor examples with automatic agitation were compared to incubator examples that were manually agitated. The control or comparative incubator was either BINDER incubator KT115 or BD115.

The examples using the inventive reactor or the comparative incubator were taken from different enzymatic reaction steps in closed linear DNA (clDNA) manufacture: (i) Phi29 polymerase for Rolling Circle Amplification (RCA), (ii) Restriction Enzymes (RE) to break up the DNA concatemer, (iii) TelN protelomerase for covalent closure of both the DNA ends, and (iv) exonucleases for removing DNA impurities.

Each of these enzymatic reaction steps had different incubation times, temperatures, and agitation frequencies. In the control or comparative incubator examples, agitation was performed manually by individuals with ×10 inversions and ×10 orbital shaking of the reaction bag to ensure the homogeneity of the solution and components availability for the different enzymes.

To reproduce the manual movements in the comparative incubator, an independent program for each enzymatic reaction step was designed for the inventive reactor for automated agitation. The plate movements of the inventive reactor were designed to achieve homogeneity of the reaction in a bag, considering the solution viscosity along the process. The solution's viscosity changes along the different reaction steps. The solution's viscosity reaches its maximum at the end of the RCA step and decreases after Restriction Enzyme (RE) addition. The program conditions, such as temperature and agitation frequency, were generally based on closed linear DNA (clDNA) production.

### Example 1 -- Experiment of clDNA Yield in Reactor Automated Agitation Process during RCA Step (20 ml Scale)

Each enzymatic reaction was assessed separately. The experimental method was common in these experiments: 20 ml reactions were performed in 50 ml single-use bioprocess containers (filling ratio: 0.400) in all reactions steps including the RCA step. Each experimental condition was performed in triplicate for statistical analyses, resulting in 6 total reactions in Example 1.

To demonstrate that the inventive reactor can substitute comparative incubators with manual agitation, the RCA step was initially tested. During this assay, the yields were observed and analyzed between (1) incubating and automatedly agitating the reaction bag in the inventive reactor, and (2) agitating manually in the comparative incubator during the RCA step. The remaining enzymatic steps were not assessed in Example 1, but rather were completed using the comparative incubator with agitations performed manually. In other words, after the RCA step, the RE step, TelN protelomerase step, and exonuclease step were performed by manual agitation in the comparative incubator.

After exonucleases processing, the clDNA yield was quantified by Quantifluor^{®} dsDNA analysis and a two-tailed t-student test was calculated (95% confidence interval (CI)). The results showed that automated agitation during the RCA step did not have a statistically significant impact on the clDNA yield when compared to manual agitation using the comparative incubator. See Table 1 below.

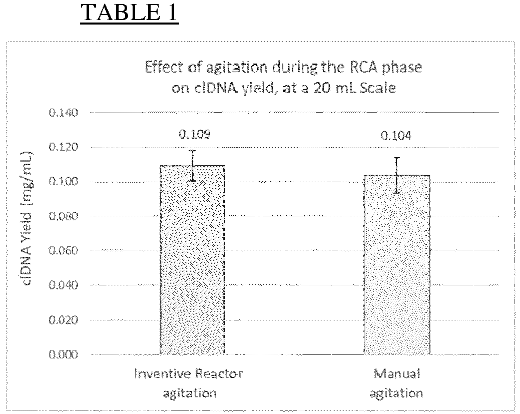

Specifically, the average clDNA yield of triplicate reactions incubated and automatedly agitated in the inventive reactor was 0.109±0.009 mg/ml, while the average clDNA yield of triplicate reactions agitated manually in the comparative incubator was 0.104±0.010 mg/ml as shown in Example 1.

### Example 2 -Experiment of clDNA Yield in Reactor Automated Agitation Process during the RCA and Restriction Enzyme Steps (20 ml Scale)

After demonstrating that automated agitation during the RCA did not have a significant effect on the clDNA yield, the inventive reactor was tested during the Restriction Enzyme (RE) step. In this assay, the yield was observed and analyzed between (1) the incubation and automated agitation in the inventive reactor during both the RCA and Restriction Enzyme (RE) steps and (2) agitating manually in the comparative incubator in these steps. The remaining enzymatic steps were not assessed in Example 2, but rather were completed using the comparative incubator with agitations performed manually. In other words, after the RCA and RE steps, the TelN protelomerase and exonuclease steps were performed by manual agitation in the comparative incubator.

The testing conditions in Example 2 included: (a) automatic agitation during the RCA and RE steps; (b) automatic agitation during the RCA step, but manual agitation during the RE step; (c) manual agitation during the RCA step, but automatic agitation during the RE step; and (d) manual agitation during the RCA and RE steps. Each condition was performed in triplicate, resulting in 12 total reactions in Example 2.

After Restriction Enzyme (RE) processing, a 0.8% agarose gel was used to check if the reactions were correctly digested. All reactions showed the expected band pattern (9005 bp, 969 bp and 860 bp bands).

The results after exonuclease processing are shown in Table 2 below. The final clDNA yield was quantified by Quantifluor^{®} dsDNA analysis and a two-tailed t-student test was calculated (95% CI). The results showed that the inventive reactor with automated agitation can substitute manual agitation in a comparative incubator during both the RCA and Restriction Enzyme (RE) steps without having a statistically significant impact on clDNA yield.

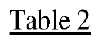

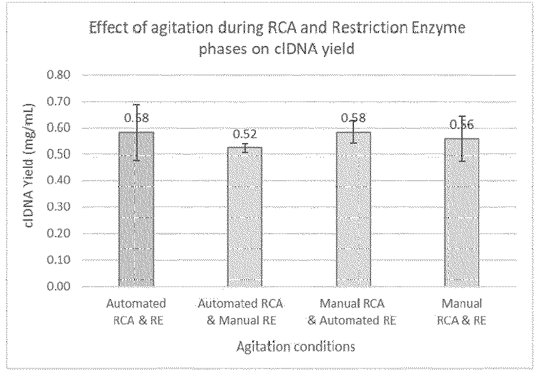

The average clDNA yield of triplicate reactions incubated and agitated in the inventive reactor was 0.58±0.11 mg/ml, while the average clDNA yield of triplicate reactions agitated manually was 0.56±0.08 mg/ml.

### Example 3 - Experiment of clDNA Yield in Reactor Automated Agitation Process (20 ml Scale) during all Enzymatic Steps

After demonstrating that automated agitation during both RCA and Restriction Enzyme (RE) steps did not have a significant effect on the clDNA yield, the inventive reactor with automated agitation was tested during the TelN and Exonuclease steps. During this assay, the yields and purities were compared between (1) incubating and automatedly agitating the reaction bag in the inventive reactor, and (2) agitating manually in the comparative incubator during the TelN and Exonuclease steps. The previous enzymatic reaction steps were not assessed in this assay, but incubated and automatedly agitated in the inventive reactor.

The testing conditions were: (a) inventive reactor incubation and automatic agitation in TelN and exonuclease steps; (b) comparative incubator using manual agitation in TelN and exonuclease steps. Each condition was performed in triplicate for each condition and construct, resulting in 12 total reactions in Example 3.

After TelN processing, two parallel 0.8% agarose gels were used to check if the two constructs were correctly processed. All reactions showed the expected band pattern after TelN processing. For the first construct, a DNA band was observed at the expected size (8884 bp) and some backbone fragments were observed (969 bp and 860 bp) as expected. For the second construct, a DNA band was observed at the expected size (4572 bp) and some backbone fragments were observed (813 bp, 799 bp, 203 bp and 201 bp) as expected.

The clDNA yields were quantified by Quantifluor^{®} dsDNA analysis and clDNA purity was analyzed by calculating the raw volume % of the clDNA by AGE densitometry. A two-tailed t-student was calculated (95% CI) in both analyses. The results showed that the inventive reactor with automated agitation can substitute for the comparative incubator with manual agitation during all the clDNA manufacturing enzymatic reactions steps without having a statistically significant impact on the clDNA yield and/or purity at a 20 ml scale. See Tables 3 and 4 below.

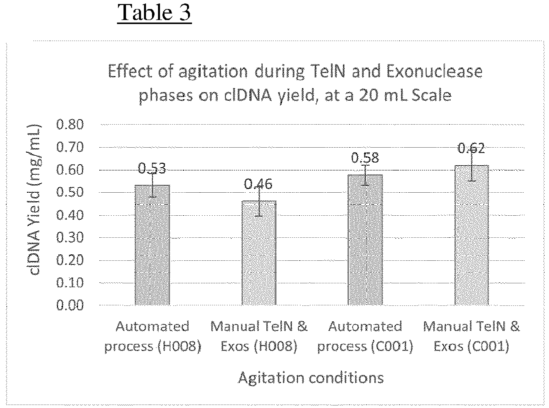

The average clDNA yield of triplicate reactions incubated and automatedly agitated in the inventive reactor during the entire process was 0.53±0.05 mg/ml and 0.58±0.04 mg/ml as shown in Table 3. The average clDNA yield of triplicate reactions agitated manually in the comparative incubator during TelN and Exonuclease steps was 0.46±0.06 mg/ml and 0.62±0.07 mg/ml as shown in Table 3.

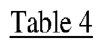

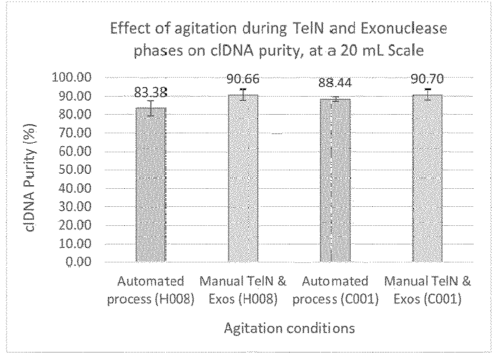

In Table 4, the average clDNA purity of triplicate reactions incubated and automatedly agitated in the inventive reactor during the entire process was 83.38±4.23% and 88.44±1.23%. The average clDNA purity of triplicate reactions agitated manually during the TelN and Exonuclease steps was 90.66±2.88% and 90.70±2.80%.

### Example 4 - Experiment of clDNA Yield and Purity in Reactor Automated Agitation Process during all Enzymatic Steps (500 ml Scale)

After demonstrating that yield and purity of clDNA manufactured with the inventive reactor at 20 ml scale reactions is maintained (Examples 1-3), an evaluation was performed by scaling-up to 500 ml scale during the reaction steps.

Two parallel 500 ml scale clDNA manufacturing batches in 1L single-use bioprocess containers (filling ratio: 0.445) were performed. The first batch was performed using the inventive reactor with automated agitation as described above. The second batch was performed using the comparative incubator that was manually agitated.

At the end of all enzymatic steps, an agarose gel was used to check if the steps were correctly processed. The automated and manual reactions showed the expected band pattern: Restriction Enzyme (RE) digested reactions completely, TelN closed the clDNA covalently, and exonucleases processed all the backbone fragments. After exonuclease processing, the final products from both reactions were analyzed to check identity and compare their final clDNA yields and purities.

The identity of the final product clDNA was confirmed by agarose gel analysis and DNA sequencing. Results from the agarose gel analysis confirmed that both reactions (inventive reactor with automated agitation and comparative incubator with manual agitation) resulted in a final product with the expected band size (4572 bp ± 10%). Sequencing analysis confirmed that both products have a 100% coincidence with the reference sequence. Overall, the results from these analyses showed that the inventive reactor with automated agitation can substitute manual agitation during all the clDNA manufacturing steps at a 500 ml scale without having an impact on the clDNA identity.

The clDNA yield was quantified by Quantifluor^{®} dsDNA analysis. The two-tailed t-student test (95% CI) was used and comparison between both reactions showed that the inventive reactor agitation with automated agitation can substitute manual agitation during all the clDNA manufacturing enzymatic process at a 500 ml scale without having a significant impact on the clDNA yield. See Table 5 below.

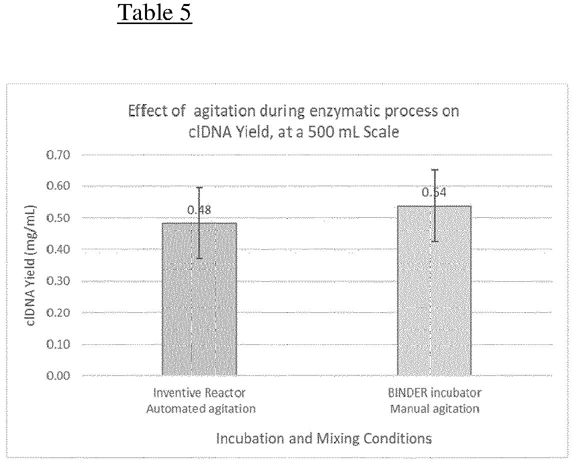

The clDNA yield of reaction incubated and agitated in the inventive reactor during the total process resulted in 0.48 mg/ml. The clDNA yield of reaction incubated in the control incubator and agitated manually during all enzymatic steps resulted in 0.54 mg/ml.

The clDNA purity was analyzed by calculating the raw volume % of the clDNA by AGE densitometry and a two-tailed t-student test (95% CI) was calculated. For an in-depth clDNA purity analysis, qPCR testing was performed. In summary, comparison of the AGE densitometry and qPCR results between both reactions (inventive reactor with automated agitation and control incubator with manual agitation) showed that the inventive reactor with automated agitation can substitute manual agitation during all the clDNA manufacturing enzymatic steps at a 500 ml scale without having a significant impact on the clDNA purity. See Table 6 below.

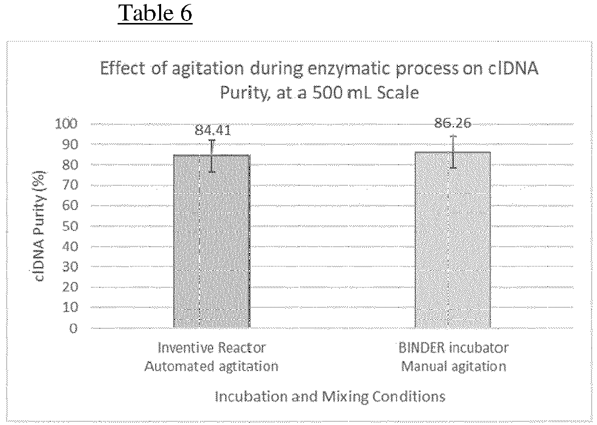

The clDNA purity of reaction incubated and agitated in the inventive reactor during the total process was 84.41 %. The clDNA purity of reaction incubated in the control incubator and agitated manually during all enzymatic steps was 86.26%.

In conclusion, the inventive reactor incubation with automated agitation can substitute manual agitation during all the clDNA manufacturing enzymatic reaction steps at a 20 ml scale, without having a statistically significant impact on the clDNA identity, yield or purity. The inventive reactor incubation with automated agitation can substitute manual agitation during all the clDNA manufacturing enzymatic reaction steps at a 500 ml scale without having a statistically significant impact on the clDNA identity, yield or purity.

While the foregoing written description of the invention enables one of ordinary skill to make and use what is considered presently to be the best mode thereof, those of ordinary skill will understand and appreciate the existence of variations, combinations, and equivalents of the specific embodiment, method, and examples herein. The invention should therefore not be limited by the above described embodiment, method, and examples, but by all embodiments and methods within the scope and spirit of the invention.

## Claims

1. A reactor comprising:
a support base;
at least one wall;
a top cover, wherein the top cover, the support base and the at least one wall form an internal chamber therein; and
a moveable plate assembly including a moveable plate located within the internal chamber, the moveable plate being configured to move in the x-direction, y-direction, and the z-direction, the temperature of the moveable plate being controllable to a desired temperature,
wherein the internal chamber is accessible via at least one of the top cover, the support base, and the at least one wall.

2. The reactor of claim 1, wherein the top cover, the support base, and the at least one wall comprise stainless steel.

3. The reactor of claim 1, wherein the at least one wall forms a porthole for viewing into the internal chamber.

4. The reactor of claim 3, wherein the at least one wall is a plurality of walls, the plurality of walls forming a respective porthole for viewing into the internal chamber.

5. The reactor of claim 1, wherein the internal chamber is accessible by moving a portion of the at least one wall and a portion of the top cover via a hinge.

6. The reactor of claim 1, wherein the moveable plate comprises aluminum.

7. The reactor of claim 1, wherein the moveable plate comprises a plurality of layers.

8. The reactor of claim 7, wherein the moveable plate comprises a resistive heating element layer.

9. The reactor of claim 1, wherein the maximum inclination of the moveable plate is about 20 degrees in any direction.

10. The reactor of claim 9, wherein the maximum inclination of the moveable plate is about 30 degrees in any direction.

11. The reactor of claim 1, wherein the movement of the moveable plate uses linear movement, rotary movement and orbital movement.

12. The reactor of claim 1, wherein the reactor has a width of from about 500 mm to about 1,500 mm, a height of from about 500 mm to about 2,000 mm, and a depth of from about 500 mm to about 1,500 mm.

13. The reactor of claim 1 further including filters to assist in preventing or inhibiting cross-contamination.

14. The reactor of claim 1, wherein the at least one wall is a plurality of walls.

15. The reactor of claim 1, wherein the internal chamber is hermetically closed.

16. A method of using a reactor for an enzymatic reaction, the method comprising:
providing a reactor of claim 1;
placing a bag or package on the moveable plate in a secured position, the bag including at least one reaction component and an enzyme;
heating the moveable plate to a desired temperature;
controlling the temperature within the internal chamber; and
moving the moveable plate in the x-direction, y-direction and the z-direction so as to mix the at least one reaction component and the enzyme.

17. The method of claim 16, wherein the bag is from about 1 to about 20 liters.

18. The method of claim 16, wherein the at least one reaction component is a nucleic acid.

19. The method of claim 18, wherein the nucleic acid is DNA and/or RNA polymerization.

20. The method of claim 16, wherein the at least one reaction component and an enzyme involves restriction endonucleases; nucleic acid; amino acid; ligase; telomerase or protelomerase; deoxynucleoside triphosphate (dNTPs); endonuclease and/or exonuclease; DNA polymerase or any variant thereof; reverse transcriptase; recombinase, protease; nuclease; Taq DNA polymerase,; phosphorylase; phosphotransferase; dephosphorylase; nucleic acid synthase; RNA polymerase; or any combination thereof.

21. The method of claim 16, wherein the enzymatic reaction is restriction digestion with restriction endonucleases; nucleic acid ligation; reaction with telomerase or protelomerase; reaction with endonuclease and/or exonuclease; reaction with DNA polymerase or any variant thereof; reaction with reverse transcriptase; reaction with recombinase, reaction with protease; reaction with nuclease; polymerase chain reaction, nucleic acid amplification reaction; phosphorylation reaction; dephosphorylation reaction; nucleic acid synthesis reaction; reaction with RNA polymerase; reaction of nick or damage repair of nucleic acid; nucleic acid denaturation reaction; nucleic acid annealing reaction; nucleic acid extension reaction; or any combination thereof.
